# EUROPÄISCHE PATENTSCHRIFT

(11) **EP 0 648 510 B1**
(45) Veröffentlichungstag und Bekanntmachung des Hinweises auf die Patenterteilung: **18.11.1998**
(21) Anmeldenummer: 94890154.1
(22) Anmeldetag: 26.09.1994
(51) Int. Cl.: A61M 5/178, B65D 81/32, A61M 5/19, A61M 5/50

(54) **Spritzenvorrichtung zur Aufbewahrung und Applikation eines Mehrkomponentenmaterials sowie Herstellungsverfahren**
Syringe device for storing and application of a multi-component material and process for manufacture thereof
Dispositif de seringue pour stockage et application d'un matériau à composants multiples et procédé de fabrication

(30) Priorität: 18.10.1993 AT 2085/93
(43) Veröffentlichungstag der Anmeldung: 19.04.1995
(73) Patentinhaber: IMMUNO Aktiengesellschaft, A-1221 Wien (AT)
(72) Erfinder: Barta, Helmut, A-1100 Wien (AT); Eder, Helmut, A-1190 Wien (AT); Granser, Manfred Ing., A-1220 Wien (AT); Habison, Georg, Dr., A-1040 Wien (AT); Hantak, Edith, Dr., A-2111 Seebarn (AT); Moser, Franz, A-2232 Deutsch Wagram (AT); Pfaffenbichler, Peter, Dipl.-Ing., A-7141 Podersdorf (AT)
(74) Vertreter: Weinzinger, Arnulf, Dipl.-Ing.

(56) Entgegenhaltungen:
- EP-A- 0 212 798
- EP-A- 0 227 401
- EP-A- 0 255 369
- EP-A- 0 294 672
- EP-A- 0 425 686
- EP-A- 0 485 028
- WO-A-92/15345
- FR-A- 2 399 861
- FR-A- 2 668 060
- US-A- 3 885 562
- US-A- 4 260 077
- US-A- 4 639 250
- US-A- 4 687 467
- US-A- 4 874 368

## Beschreibung

Die Erfindung betrifft eine Spritzenvorrichtunng gemäß dem einleitenden Teil von Anspruch 1, zur Aufbewahrung und Applikation eines biologischen Mehrkomponentenmaterials, vorzugsweise eines Gewebeklebstoffes auf Basis von menschlichen oder tierischen Proteinen zum nahtlosen bzw. nahtunterstützenden Verbinden von menschlichen oder tierischen Gewebe- oder Organteilen, zur Wundversiegelung, Blutstillung und dergl.

Weiters betrifft die Erfindung ein Verfahren zum Herstellen einer befüllten, sterilen Spritzenvorrichtung gemäß dem einleitenden Teil von Anspruch 21.

Applikationsgarnituren mit einer Spritzenvorrichtung, mit einer gemeinsamen Betätigungseinrichtung für die Kolben aller Spritzenkörper, und mit einem Abgabeteil, etwa in Form eines Sprühkopfes oder eines Anschlußkopfes, an dem ein Sprühkatheter befestigt wird, oder einer Mischkanüle, sind beispielsweise aus der EP 37 393 A (bzw. der entsprechenden US 4 359 049 A), der EP 156 098 A (bzw. der entsprechenden US 4 631 055 A) sowie der EP 210 160 A (bzw. der entsprechenden US 4 735 616 A) bekannt. Ähnliche Applikationsgarnituren sind in der US 5 116 315 A und FR 2 661 097 A geoffenbart. Derartige Garnituren werden insbesondere zur Applikation eines Gewebeklebstoffes eingesetzt, welcher durch Zusammenbringen mit blutgerinnungsfördernden Gerinnungsenzymen in situ verfestigt wird, wobei als Komponenten einerseits eine Faktor XIII und Fibrinogen enthaltende Proteinlösung (Gewebeklebstoff) und andererseits eine Thrombin enthaltende Lösung verwendet werden. Diese Komponenten werden im Anwendungsfall über den mit den Spritzenkörpern der Spritzenvorrichtunng durch Aufstecken auf deren Spritzenkonusse verbundenen Abgabeteil - etwa den Anschlußkopf mit Mischkanüle oder den Sprühkopf - auf die gewünschte Stelle, z.B. eine zu behandelnde oder zu schützende Wundstelle, aufgebracht.

Bei den bekannten Garnituren ist zur Verbindung der Spritzenkörper eine allgemein wannen- oder hülsenförmige Halteeinrichtung mit entsprechenden Wannen oder Rinnen für die Aufnahme der Spritzenkörper vorgesehen, wobei diese Halteeinrichtung weiters mit in entgegengesetzten Richtungen seitlich abstehenden Fingergriffen versehen ist. In diese Halteeinrichtung werden die Spritzenkörper eingesetzt, wobei z.B. elastisch ausweichende Schnappvorsprünge die Spritzenkörper festhalten. Zur Betätigung der Kolben der Spritzenkörper werden die mit den Kolben fest verbundenen Kolbenstangen weiters mit einem gemeinsamen Griffteil verbunden, und es wurde auch vorgeschlagen, zur Stabilisierung bzw. Verbesserung der Führung der Kolbenstangen bei der Betätigung der Spritzenvorrichtung eine Führungsstange mit dem gemeinsamen Griffteil zu verbinden; diese Führungsstange erstreckt sich durch eine Führungsbohrung in der Haltevorrichtung.

Mit einer derartigen Ausbildung wird im Vergleich zu früheren Ausbildungen, etwa gemäß US 3 223 083 A oder US 2 112 160 A, bereits ein wesentlicher Fortschritt hinsichtlich einer einfachen Montage sowie einer größeren Sicherheit bei der Handhabung erzielt, da einerseits die Spritzenkörper in die Halteeinrichtung z.B. eingeschnappt werden können, und andererseits die Kolbenstangen zur gemeinsamen Betätigung der Kolben miteinander verbunden und überdies bei der Bewegung durch die Führungsstange geführt werden, so daß eine gleichmäßige Betätigung der Kolben aller Spritzenkörper sichergestellt ist. Demgegenüber sind bei der Vorrichtung gemäß der US 2 112 160 A die Spritzenkörper, die längsseitig unmittelbar aneinander anliegen, in aufwendiger Weise durch eine Zementmasse sowie durch Drahtschlingen und Gummibänder miteinander verbunden. Bei der Vorrichtung gemäß US 3 223 083 A sind die Spritzenkörper durch eine sie an einer mittleren Stelle umschließende Klemme miteinander verbunden, wobei die Spritzenkörper keine stabile Lage relativ zueinander einnehmen. Außerdem müssen hier die beiden Kolbenstangen vom Benützer gemeinsam und möglichst gleichmäßig vorgeschoben werden, wozu eine große Geschicklichkeit erforderlich ist.

Es hat sich nun gezeigt, daß auch die Spritzengarnituren der hier in Rede stehenden Art, z.B. gemäß EP 37 393 A oder EP 156 098 A, noch insofern nachteilig sind, als die Montage der Spritzenkörper in der Halteeinrichtung ebenso wie die Verbindung der Kolbenstangen über den gemeinsamen Griffteil praktisch nicht maschinell durchgeführt werden kann, sondern bloß händisch, wo-durch die sterile Arbeitsweise erschwert wird bzw. zusätzliche Sterilisationsbehandlungen notwendig sind. Ein steriles Befüllen ist nur mit großem Aufwand möglich.

Wünschenswert ist es nun, eine sterile bzw. aseptische Behandlung der Garniturteile und insbesondere der Spritzenvorrichtung während des gesamten Fertigungs-, Befüllungs- und Verpackungsprozesses bis hin zur Inbetriebnahme in einfacher Weise zu ermöglichen. Diese aseptische bzw. sterile Fertigung, Befüllung und Verpackung ist dabei insbesondere im Hinblick auf die speziellen Komponenten für den bevorzugten Einsatz der Applikationsgarnitur, nämlich zum Aufbringen eines Gewebeklebstoffes, von ganz besonderer Bedeutung.

Darüber hinaus ist bei den bekannten Applikationsgarnituren insofern noch ein relativ hoher Fertigungsaufwand erforderlich, als abgesehen von den Spritzenkörpern für die Spritzenvorrichtung und den Kolben mit Kolbenstangen und Betätigungseinrichtung mehrere gesonderte Bauteile, nämlich die Halteeinrichtung einerseits sowie der gemeinsame Griffteil samt Führungsstange andererseits, hergestellt werden müssen, wobei diese Bauteile sodann mit den entsprechenden Teilen - händisch - verbunden werden müssen.

Es wurden andererseits bereits Doppelspritzen vorgeschlagen (vgl. US 3 828 980 A und US 4 040 420 A), bei denen die Spritzenzylinder direkt längsseitig aneinander angeformt sind, so daß sich insofern im Vergleich zu den vorstehenden Spritzengarnituren in der Fertigung und Handhabung eine Vereinfachung ergibt. Von Nachteil ist hier allerdings, daß das Befüllen der Spritzenzylinder mit den unterschiedlichen Komponenten - die vor der Applikation nicht miteinander in Kontakt gelangen dürfen - wegen der unmittelbaren Nachbarschaft der Spritzenzylinder ohne gegenseitige Kontamination besonders schwierig ist. Darüber hinaus ist auch die gemeinsame Betätigung der beiden Kolben schwierig.

In der FR 2 668 060 A ist eine Spritzenvorrichtung mit mehreren Spritzenkörpern gezeigt, die in einem Wannenteil eingesetzt werden, der als Verbindungsteil zu einer Halte- und Betätigungseinrichtung dient, und der Ausnehmungen bzw. Schlitze zum Aufstecken einer Gas-Zusatzeinheit aufweist. Dabei ist in bloß ganz allgemeiner Form auf die Möglichkeit einer einstückigen Ausbildung der Spritzenkörper hingewiesen, jedoch wird deren gesonderte Fertigung als bevorzugt herausgestellt.

Die EP 294 672 A offenbart eine Doppel-Austragkartusche für Zweikomponentenmassen, wie Kleber, Dichtmassen, Dental-Abdruckmassen, wo eine Sterilität nicht im Vordergrund steht, und wobei die getrennten Vorratszylinder entweder über eine Schnappverbindung oder dergl. oder aber einstückig über vordere und hintere Steqe miteinander verbunden sind.

Eine andersartige Doppelsprltze, mit zwei gesonderten Injektionsnadeln, ist schließlich aus der US 3 552 394 A bekannt, wobei hier die beiden Spritzenkörper über einen in der Dicke abgestuft ausgebildeten Verbindungsteil zu einer einstückigen Spritzeneinheit zusammengefaßt sind, in die die Spritzenkammern gebohrt werden. Dabei wird angestrebt, den von einem Patienten empfundenen Schmerz dadurch zu reduzieren, daß die beiden Injektionsnadeln relativ eng nebeneinander angeordnet werden, so daß beim Einstecken beider Nadeln nur ein einziger Einstechschmerz verspürt wird. Auch hier ist u.a. nachteilig, das ein getrenntes Befüllen beider Spritzenampullen mit verschiedenen Substanzen kritisch ist.

Es ist daher Aufgabe der Erfindung, hier Abhilfe zu schaffen und eine Spritzenvorrichtung der eingangs angeführten Art vorzusehen, wobei einerseits eine aseptische Behandlung während der gesamten Fertigung, Füllung und Verpackung der Spritzenvorrichtung sichergestellt und eine unabhängige, nachträgliche Sterilisierung der übrigen Garniturteile durchgeführt werden kann, und andererseits eine einfache Konstruktion erzielt wird, die ohne gesonderte, zusätzliche Bauteile auskommt, und wobei überdies eine einfache und sichere Betätigung der Kolben aller Spritzenkörper gewährleistet sein soll. Weiters soll ein Verfahren vorgesehen werden, mit dem bei der Herstellung bzw. Befüllung der Spritzenvorrichtung auf rationelle Weise die Sterilität sichergestellt werden kann.

Zur Lösung der vorstehenden Aufgabe weist die erfindungsgemäße Spritzenvorrichtung die in Anspruch 1 angegebenen Merkmale auf.

Das erfindungsgemäße Verfahren enthält in entsprechender Weise die in Anspruch 21 angegebenen Merkmale.

Vorteilhafte Ausgestaltungen und Weiterbildungen sind Gegenstand der abhängigen Ansprüche.

Mit den angegebenen Maßnahmen wird der vorstehenden Zielsetzung in vorteilhafter Weise entsprochen. Die Spritzenvorrichtung wird durch eine kompakte, stabile Spritzeneinheit gebildet, der eine ebenso kompakte, stabile Kolbenstangeneinheit zugeordnet ist. Beide Einheiten können dabei problemlos völlig automatisch hergestellt, d.h. einstückig geformt, werden, was vor allem im Fall der Spritzeneinheit im Hinblick auf die gewünschte Sterilität und "Partikelfreiheit" besonders wesentlich ist. Im Fall der Kolbenstangeneinheit ist auch, ähnlich wie für den Abgabeteil, eine nachträgliche Sterilisation im bereits verpackten Zustand problemlos möglich, d.h. es ist hier nicht notwendig, die Kolbenstangeneinheit im Sterilbereich herzustellen oder in die Spritzeneinheit einzusetzen. Die Kolbenstangeneinheit kann auch zusammen mit dem Abgabeteil (z.B. einem Anschlußstück mit getrennten Kanälen zum Fördern der einzelnen Komponenten, gegebenenfalls mit einer Mischkanüle) in einer gemeinsamen Verpackung verpackt und nachträglich einer Sterilisationsbehandlung unterworfen werden.

Es ist somit für die Spritzeneinheiten in vorteilhafter Weise möglich, alle Vorgänge von der Fertigung an, bevorzugt durch Spritzgießen, über ein etwaiges zwischendurch erfolgendes Verpacken und Auspacken der Spritzeneinheit, über ein Vereinzeln der Spritzeneinheiten zum maschinellen Befüllen der Spritzenkörper bis zu deren Verschließen mittels der Kolbenstopfen und Verpacken, aseptisch ablaufen zu lassen, wobei keinerlei händische Eingriffe erforderlich sind. Dies ist vor allem im Hinblick auf die bevorzugt verwendeten, zu applizierenden Materialien, nämlich Proteinlösungen etc., von ganz wesentlichem Vorteil. Die erfindungsgemäße Spritzenvorrichtung, noch leer oder bereits befüllt, zeichnet sich somit in jedem Stadium dadurch aus, daß sie steril ist.

Beim maschinellen Befüllen der Spritzenkörper wird zur Verhinderung einer gegenseitigen Kontaminierung mit den Komponenten oder Reaktionsflüssigkeiten ein Schutzteil, beispielsweise in Form eines Abdeckwinkels, vorgesehen, der in die Aufnahme-Ausnehmung zwischen den (jeweiligen) Spritzenkörpern hineinragt und dabei den oder die übrigen Spritzenkörper von dem gerade befüllten Spritzenkörper trennt und abschirmt. Im Prinzip ist es dabei aber auch denkbar, ein Schutzblech oder dergl. Schutzteil derart zwischen die Spritzenkörper in die Aufnahme einzuführen, daß die Spritzenkörper beidseits dieses Schutzteils gleichzeitig befüllt werden, wobei auch in diesem Fall eine gegenseitige Kontaminierung durch diesen Schutzteil wirksam verhindert wird. Somit trägt dieses Merkmal der Aufnahme-Ausnehmung zwischen den Spritzenkörpern für den beim Befüllen einzuführenden Schutzteil wesentlich zur Möglichkeit einer sterilen maschinellen Verfüllung bei, wobei nichtsdestoweniger die einfache einstückige, kompakte Bauweise der Spritzeneinheit gewährleistet ist.

Zweckmäßigerweise können bzw. kann die Spritzeneinheit, die Kolbenstangeneinheit und/oder der Abgabeteil zumindest bereichsweise, z.B. in Form eines Streifens, röntgenstrahlen-absorbierendes Material aufweisen; beispielsweise kann ein Metallstreifen mit eingeformt werden. Auf diese Weise ist eine etwaige spätere Ortung mittels einer Röntgeneinrichtung möglich.

Ferner ist es, vor allem aus Sicherheitsgründen, etwa um zu verhindern, daß ein Arzt beim Gebrauch irrtümlich die Kolbenstopfen zurückzieht, von Vorteil, wenn die Kolbenstopfen in den Spritzenkörpern mittels der Kolbenstangeneinheit nur in Richtung der Konusse bewegbar sind. Dies kann beispielsweise dadurch bewerkstelligt werden, daß der reibungsschlüssige Sitz der Kolbenstopfen in den Spritzenkörpern fester ist als ein beispielsweise vorgesehener Schnappsitz der Kolbenstangen in hinteren Öffnungen der Kolbenstopfen im Fall einer Zugbeanspruchung.

Besonders zweckmäßig ist es jedoch, wenn die Kolbenstangen einfach an der Rückseite der Kolbenstopfen anliegen, wobei ein leichter reibungsschlüssiger Halt für die Kolbenstangen in den Spritzenkörpern, etwa durch abstehende Flügel, vorgesehen werden kann, um ihr Herausfallen beim Gebrauch zu verhindern. Die gesonderten Kolbenstopfen können zur Gänze aus Silikonmaterial bestehen, was im Hinblick auf die gewünschten Gleiteigenschaften über lange Zeiträume (beispielsweise 2 Jahre) und auf die erforderliche Reinheit (wobei z.B. kein Gleitmittel verwendet werden darf) von Vorteil ist.

In einer bevorzugten Ausführungsform werden Kolbenstopfen verwendet, die aus vollem Material bestehen und kugelförmig ausgebildet sind. Derartige Kolbenstopfen weisen nur eine Dichtlippe auf, was das Vorschieben der Kolbenstopfen in Richtung der Konusse erleichtert. Ein weiterer Vorteil dieser Ausführungsform besteht darin, daß nach Abfüllen der Spritzenkörper die Kolbenstopfen in einfacher Weise eingesetzt werden können, da auf eine besondere Ausrichtung der Kolbenstopfen nicht geachtet werden muß.

Bei Verwendung von Kolbenstangen mit entsprechender Ausnehmung ist ein einfaches Bewegen dieser kugelförmigen Kolbenstopfen unter Erhaltung der Dichtheit nur in Richtung der Konusse durch Druck der Kolbenstangen möglich. In diesem Fall sind die Konusse entsprechend konkav ausgebildet, um ein vollständiges Entleeren der Spritzenkörper zu gewährleisten.

Es sei hier noch erwähnt, daß aus der US-5 147 323 A eine Ampulleneinheit mit mehreren in einem schachtelförmigen Behälter nebeneinander angeordneten Ampullen bekannt ist, die rückseitig durch einfache, lose Kolberstopten abgeschlossen sind; je nach Stellung eines den Behälters verschließenden Schiebedeckels, der eine Öffnung zum Einführen einer Kolbenstange aufweist, kann in einer der Ampullen der Kolbenstopfen vorwärts gedrückt werden, um letztlich die Abgabe einer Dosis Insulin über eine ausschiebbare Nadel zu bewirken. Diese bekannte Ampulleneinheit ist insgesamt äußerst komplex und aufwendig und für die Applikationen eines Gewebeklebstoffes kaum geeignet.

Weiters ist aus der US 4 639 250 A eine Spritze bekannt, deren Spritzenzylinder bei der Herstellung in einer Einheit mit anderen Spritzenzylindern erzeugt wird, bei der die Spritzenzylinder im Bereich ihrer Konusse untereinander durch Stege verbunden sind; diese Stege werden nachfolgend durchtrennt, um Einzelspritzen zu erhalten.

Zur Erzielung einer besonders hohen Festigkeit der Spritzeneinheit gegenüber Verwinden oder Bruch hat es sich als besonders vorteilhaft erwiesen, wenn als Verbindungsteil zumindest zwei zueinander parallele, plattenförmige Verbindungsstege zwischen den durch sie einstückig verbundenen Spritzerkörpern verlaufen. Dabei ist es weiters gurstig, wenn jeder Verbindungssteg tangential an den jeweiligen Spritzerkörper anschließt. Ganz allgemein kann die Spritzeneinheit zwecks einfacher Handhabung flach und plattenförmig, mit einer Dicke entsprechend der Dicke bzw. dem Außendurchmesser der Spritzenkörper, ausgebildet sein. Eine derartige flache, kompakte, plattenförmige Spritzeneinheit ist selbstverständlich für jegliche Art von Manipulation günstig, abgesehen davon, daß diese Spritzeneinheit eine besonders hohe Bruchfestigkeit besitzt. Weiters können die Verbindungsstege mit zur Wandstärke der Spritzenkörper vergleichbaren Dicken problemlos hergestellt werden, so daß auch bei der Herstellung durch Gießen oder dergl. beim Abkühlen keine Schwierigkeiten auftreten.

Aus der EP-210 160 A (bzw. der entsprechenden US-4 735 616 A) ist es bereits bekannt, die Spritzenkörper mit unterschiedlichen Querschnitten, bei gleicher Kolben-Hublänge, vorzusehen, um so unterschiedliche, vom Verhältnis 1:1 abweichende Mischungsverhältnisse der Proteinlösung und der Thrombinlösung im Fall der Applikation eines Gewebeklebstoffes zu ermöglichen. Bei der bekannten Vorrichtung werden entsprechend dimensioinerte kreiszylindrische Spritzenkörper in den - entsprechend bemessenen - Wannen der Halteeinrichtung eingeschnappt. Bei der zuletzt genannten Ausführungsform der erfindungsgemäßen Vorrichtung ist nun trotz des Umstandes, daß eine flache plattenförmige Ausbildung der gesamten Spritzeneinheit vorliegt, ebenfalls ein unterschiedliches Mischungs- bzw. Lösungsverhältnis möglich. Eine vorteilhafte Ausführungsform der erfindungsgemäßen Vorrichtung ist demgemäß dadurch gekennzeichnet, daß bei Vorsehen von Spritzenkörpern mit unterschiedlichen Querschnittsflächen und konstanter Kolben-Hublänge die Querschnittsfläche zumindest eines Spritzenkörpers oval ist, wobei ihre Abmessung quer zur Dickenrichtung der plattenförmigen Spritzeneinheit von jener der Querschnittsfläche des oder der anderen Spritzenkörper verschieden ist.

Eine herstellungstechnisch besonders einfache Ausbildung wird andererseits dann ermöglicht, wenn als Verbindungsteil wenigstens ein sich plattenförmig entsprechend einer durch die Längsachsen der Spritzenkörper definierten Mittenebene erstrekkender und mittig an den jeweiligen Spritzenkörper anschließender Verbindungssteg vorgesehen ist.

Um einerseits eine gegenseitige Kontaminierung ausreichend wirksam zu verhindern und andererseits die Festigkeit der Verbindung der Spritzenkörper durch den oder die Verbindungsstege nicht durch eine zu tiefe Aufnahme-Ausnehmung zu beeinträchtigen, hat es sich bei den gängigen Spritzendimensionen als besonders vorteilhafter Kompromiß erwiesen, wenn der (jeweilige) Verbindungsteil zur Belassung der Aufnahme-Ausnehmung in einem Abstand von zumindest 5 mm, vorzugsweise in einem Abstand von 5 mm bis 15 mm, insbesondere ca. 10 mm, vom hinteren Ende der Spritzeneinheit endet.

Wie erwähnt sind bei den bekannten Spritzenvorrichtungen der hier in Rede stehenden Art Fingergriffe an der wannenförmigen Halteeinrichtung vorgesehen, die relativ zu den Spritzenkörpern seitlich und in entgegengesetzten Richtungen von der Halteeinrichtung abstehen. Fingergriffe können selbstverständlich auch bei der erfindungsgemäßen Spritzenvorrichtung vorgesehen werden, wobei es zweckmäßig ist, die Fingergriffe einfach direkt an den hinteren Enden der Spritzenkörper anzuformen. Die Fingergriffe können dabei eine Breite vergleichbar dem Außendurchmesser der Spritzenkörper haben, so daß sie im Falle der besonders bevorzugten flachen, plattenförmigen Ausbildung der Spritzeneinheit innerhalb der Dickenabmessung der Spritzeneinheit liegen.

An sich wäre es denkbar, die Spritzenkörper und den oder die Verbindungsstege miteinander etwa durch Verschweißen, etwa Ultraschallschweißen, fest und einstückig zu verbinden. Besonders bevorzugt wird es jedoch im Hinblick auf eine einfache Herstellung sowie eine einheitliche kompakte Vorrichtung, wenn die Spritzeneinheit ein einstückiger Polypropylen-Spritzgußteil ist. Polypropylen eignet sich dabei insbesondere für die Herstellung der Spritzenkörper, da es keine sog. "Auslöse"eigenschaft hat, d.h. es gehen keine Bestandteile, wie Weichmacher etc., aus dem Material in den Spritzeninhalt, d.h. in das in den Spritzenkörpern aufgenommene jeweilige Produkt, über.

Auch ist es von Vorteil, wenn der bzw. zumindest ein Verbindungsteil wenigstens ein Beschriftungsfeld aufweist. Dabei kann auf diesem Beschriftungsfeld vor bzw. nach dem Befüllen der Spritzenkörper eine Beschriftung, etwa betreffend den Inhalt der Spritzenkörper, die Applikationsweise etc., angebracht werden. In diesem Zusammenhang ist es denkbar, zur Beschriftung direkt einen Aufdruck auf dem Beschriftungsfeld vorzusehen, oder aber es kann ein Beschriftungsetikett oder dergl. aufgebracht, z.B. aufgeklebt werden. Möglich wäre es auch, beim Formen der Spritzeneinheit eine Beschriftung durch Prägung anzubringen, wobei eine derartige reliefartige Prägestruktur auch eine gewisse Versteifungswirkung für den Verbindungssteg hätte. An sich können aber selbstverständlich auch unabhängig davon Versteifungsrippen oder dergl. an den Verbindungsstegen angeformt werden.

Bei der Betätigungseinrichtung ist es ferner von besonderem Vorteil, wenn zur zusätzlichen Versteifung der Kolbenstangeneinheit bzw. zur Erhöhung der Festigkeit der Verbindung zwischen den Kolbenstangen an den Griffteil anschließend ein sich zwischen den Kolbenstangen erstreckender, mit ihnen fest verbundener Versteifungssteg vorgesehen ist.

Auch hier ist es im übrigen wiederum bevorzugt, wenn die Kolbenstangeneinheit ein einstückiger Spritzgußteil ist.

Die Betätigungseinrichtung bzw. Kolbenstangeneinheit wird erst zu einem späteren Zeitpunkt, nach dem Befüllen der Spritzenkörper und deren Verschließen durch Einsetzen der Kolbenstopfen, in die Spritzenvorrichtung eingesetzt, und zwar insbesondere erst unmittelbar vor dem Gebrauch. Dabei ergibt sich, daß die Kolbenstangeneinheit mit den in den Spritzenkörpern enthaltenen Produkten nicht direkt in Kontakt kommt. Demgemäß ist die Materialwahl für die Kolbenstangeneinheit nicht so kritisch, und es kann hier das Material in erster Linie im Hinblick auf die gewünschte einfache Herstellung sowie die angestrebte hohe Festigkeit der Kolbenstangeneinheit ausgewählt werden. In diesem Zusammenhang hat es sich als besonders vorteilhaft erwiesen, wenn die Kolbenstangeneinheit aus Acrylnitril-Butadien-Styrol-Copolymer (ABS) besteht.

Beim Herstellen der befüllten Spritzenvorrichtung unter Einhaltung der Sterilität ist es auch von besonderem Vorteil, wenn die Spritzenvorrichtung unmittelbar nach dem Befüllen mit den Komponenten und Verschließen mit den Kolbenstopfen steril verpackt wird. Weiters ist es günstig, wenn jeweils die einander entsprechenden Spritzenkörper mehrerer in einer Reihe angeordneter Spritzeneinheiten gleichzeitig unter Einführung (jeweils) eines gemeinsamen Schutzteiles in die Ausnehmungen dieser Spritzeneinheiten steril befüllt werden.

Die Erfindung wird nun nachstehend anhand von in der Zeichnung veranschaulichten, besonders bevorzugten Ausführungsbeispielen, auf die sie jedoch nicht beschränkt sein soll, noch weiter erläutert.

Es zeigen:
Fig.1 eine schematische Ansicht einer Spritzenvorrichtung zur Applikation eines Gewebeklebstoffes mit eingesetzter Kolbenstangeneinheit;
Fig.2 eine Draufsicht auf die Spritzenvorrichtung, bei weggenommener Kolbenstangeneinheit, gemäß der Linie II-II in Fig.1;
Fig.3 eine Unteransicht der Spritzenvorrichtung von Fig.1, gemäß Pfeil III in Fig.1;
Fig.4 eine Draufsicht auf die Kolbenstangeneinheit der Vorrichtung gemäß Fig.1, gemäß Pfeil IV in Fig.1;
Fig.5 in schematischer Ansicht eine andere Ausführungsform einer Spritzenvorrichtung mit eingesetzter Kolbenstangeneinheit;
Fig.6 eine Draufsicht auf die Spritzenvorrichtung, bei entfernter Kolbenstangeneinheit, gemäß der Linie VI-VI in Fig.5;
Fig.7 eine Ansicht der Betätigungseinrichtung bzw. Kolbenstangeneinheit, wobei einer der Kolben in Explosionsdarstellung und teilweise aufgeschnitten und der andere Kolben auf die Kolbenstange aufgeschnappt veranschaulicht sind;
Fig.8 eine Draufsicht auf diese Kolbenstangeneinheit;
die Fig.9, 9A und 9B ein Schema zur Veranschaulichung des automatischen Befüllens einer Spritzeneinheit mit zwei voneinander getrennt zu haltenden Komponenten in einer Anlage, wobei Fig.9 eine schematische Draufsicht auf in einer Linie befüllte Spritzeneinheiten ist und Fig.9A bzw. 9B schematische Ansichten gemäß den Linien A-A- bzw. B-B in Fig.9 sind;
Fig.10 schematisch in einer Ansicht das Verschließen einer Spritzeneinheit mit Kolben;
Fig.11 eine Draufsicht auf die verschlossene Spritzeneinheit;
die Fig. 12 und 13 in einer Ansicht bzw. Unteransicht eine weitere, derzeit besonders bevorzugte Kolbenstangeneinheit;
Fig.14 eine Ansicht von voll ausgebildeten Kolbenstopfen zum Zusammenarbeiten mit der Kolbenstangeneinheit gemäß Fig. 12 und 13; und
Fig.15 eine Ansicht eines Abgabeteils in Form eines an sich bekannten Aufsteckkopfes mit Mischkanüle, zur Komplettierung der Darstellung der Spritzengarniturteile.

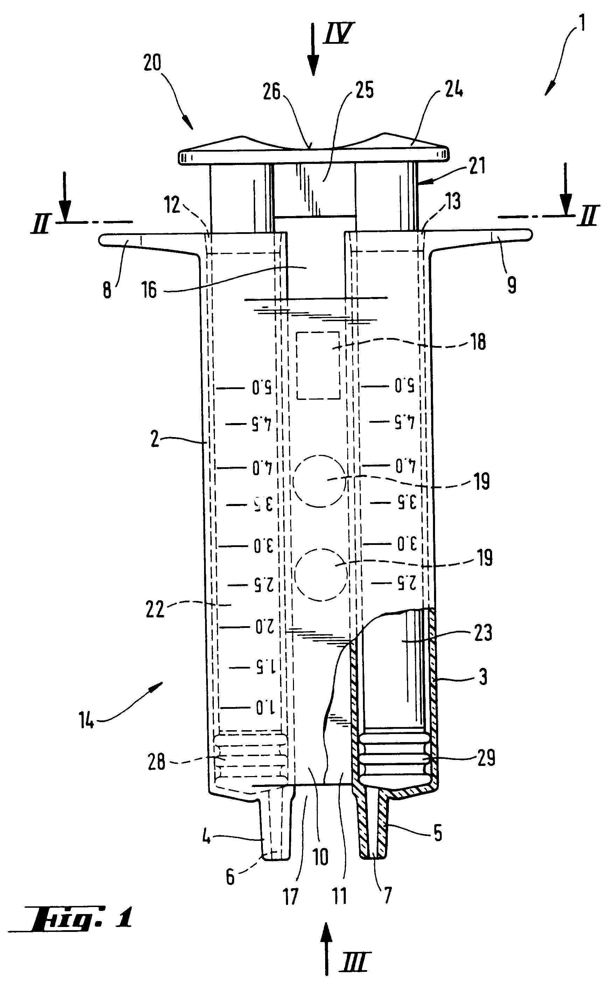

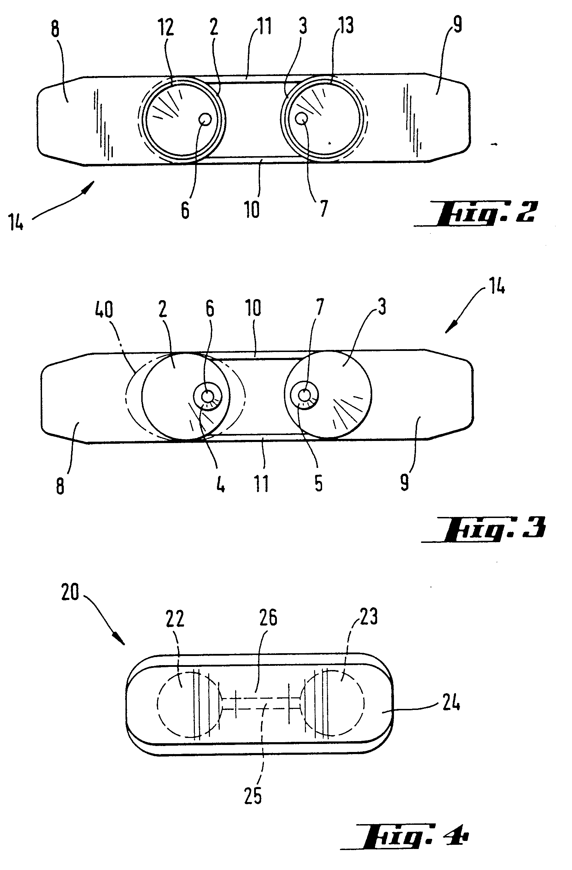

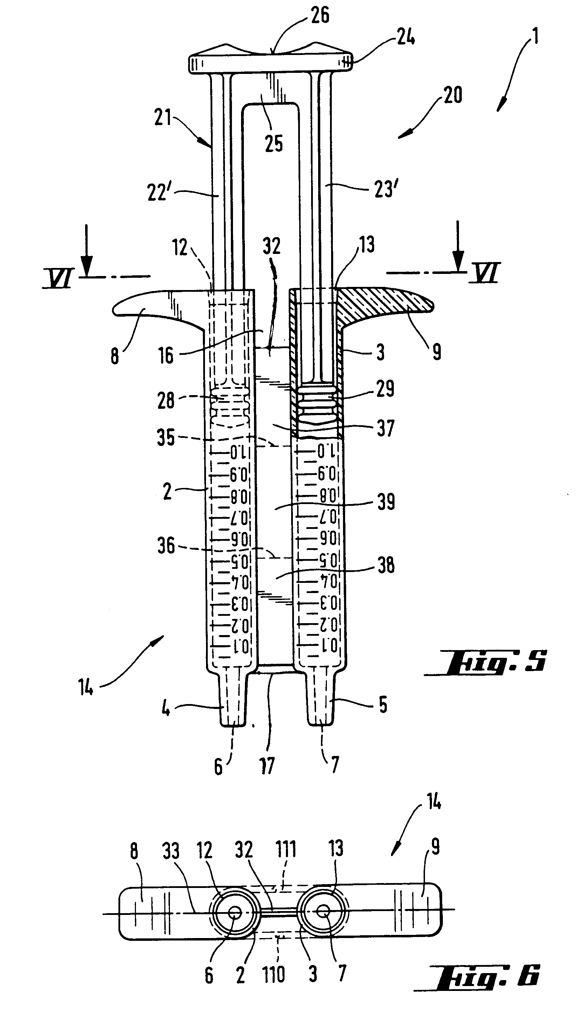

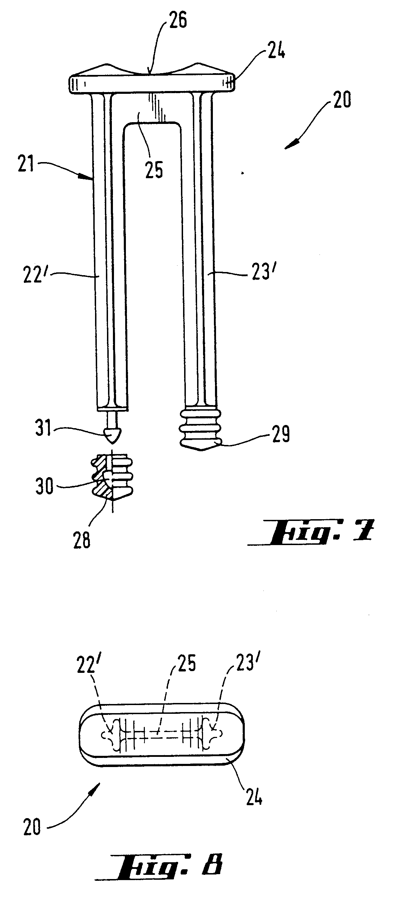

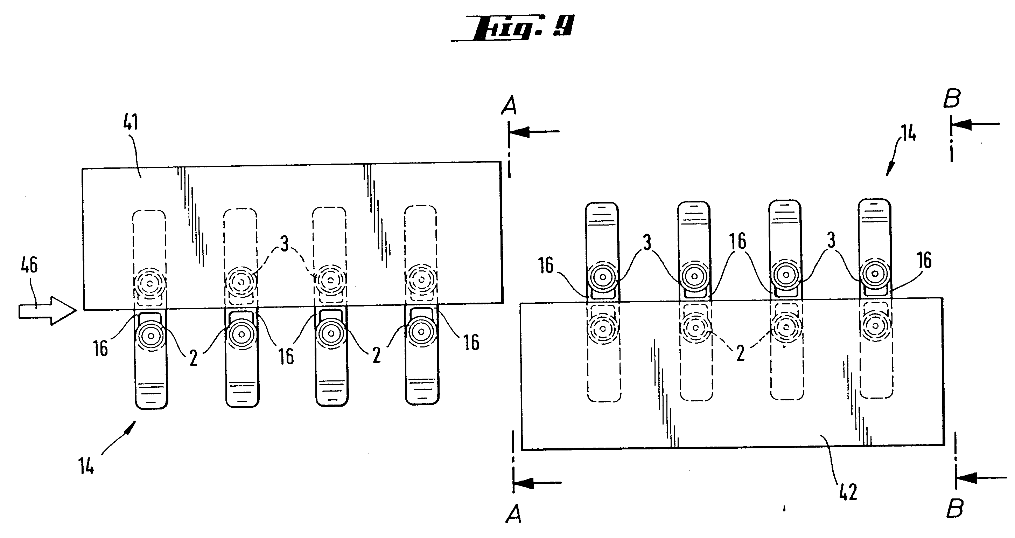

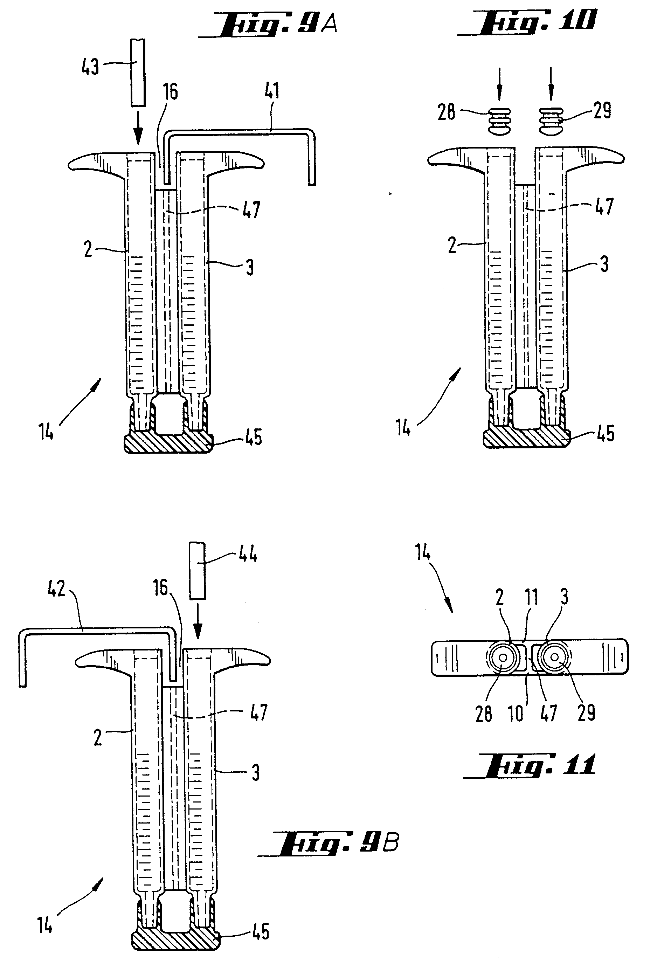

In Fig.1 ist eine allgemein mit 1 bezeichnete Spritzenvorrichtung als Teil einer Spritzengarnitur zur Aufbewahrung und Applikation eines Gewebeklebstoffes auf Basis von menschlichen oder tierischen Proteinen veranschaulicht. Die Spritzenvorrichtung 1 enthält zwei Spritzenkörper 2, 3, von denen der eine zur Aufnahme einer Thrombin enthaltenden Lösung und der zweite zur Aufnahme einer Faktor XIII und Fibrinogen enthaltenden Lösung dient. Die Spritzenkörper 2, 3 haben, wie aus Fig.2 und 3 ersichtlich ist, im gezeigten Ausführungsbeispiel kreisförmigen Querschnitt, und sie besitzen an ihren vorderen Enden in an sich herkömmlicher Weise jeweils einen Spritzenkonus 4 bzw. 5, der bis zur Ingebrauchnahme durch eine Verschlußkappe (in Fig.1 nicht dargestellt, vgl. jedoch die Kappe 45 in Fig. 9A, 9B und 10), wie an sich herkömmlich, verschlossen sein kann. Auf die Spritzenkonusse 4, 5 wird beim Gebrauch als weiterer Spritzengarniturteil ein Abgabeteil, beispielsweise ein Ansteck- oder Sammelkopf mit einer Mischkanüle (vgl. die noch zu erläuternde Fig.15), ein Anschlußstück mit einer Kanüle oder ein Sprühkopf, aufgesetzt, wie dies an sich bekannt ist.

Aus Fig.1 und 2 sind weiters die in den Spritzenkonussen 4, 5 vorgesehenen Bohrungen 6, 7 ersichtlich.

Am hinteren Ende der Spritzenkörper 2, 3 sind unmittelbar Fingergriffe 8, 9 angeformt, die in zueinander entgegengesetzten Richtungen seitlich von den Spritzenkörpern 2, 3 abstehen.

Die Spritzenkörper 2, 3 sind durch einen vorderseitigen und einen rückseitigen Verbindungssteg 10 bzw. 11 (s. auch Fig.2 und 3) zu einer festen, unzerlegbaren, kompakten, flachen, plattenförmigen Einheit miteinander verbunden. Diese Verbindungsstege 10, 11 sind mit ihrer Außenseite tangential an die Außenseite der zylindrischen Spritzenkörper 2, 3 angeschlossen, und sie erstrecken sich fast über die gesamte Länge der Spritzenkörper 2, 3, enden jedoch jeweils in einem Abstand vom vorderen Ende (Spritzenkonus-Ende) bzw. hinterem Ende (wo die Spritzenkörper 2, 3 mit einer Fase 12 bzw. 13 versehen sind), so daß sowohl am vorderen Ende als auch am hinteren Ende der durch Verbinden der Spritzenkörper 2, 3 gebildeten Spritzeneinheit 14 Ausnehmungen 16 bzw. 17 freigelassen sind, wobei die hintere Ausnehmung 16 zum Einführen eines Schutzteils, etwa in Form eines abgewinkelten Abdeckblechs, wie nachstehend anhand der Fig.9, 9A und 9B noch näher erläutern werden wird, beim maschinellen Befüllen der Spritzenkörper 2, 3 dient.

Die Verbindungsstege 10, 11 sind einstückig mit den Spritzenkörpern 2, 3 geformt, insbesondere gespritzt, wobei als Material für die einstückige Spritzeneinheit 14 vor allem Polypropylen bevorzugt wird.

Auf den Verbindungsstegen 10, 11 können, wie dies in Fig.1 mit gestrichelter Linie schematisch veranschaulicht ist, ein oder mehrere Beschriftungsfelder 18, 19 vorgesehen sein, wobei dort entweder direkt ein Aufdruck vorgenommen oder aber ein bedrucktes Etikett oder dergl. angebracht werden kann. Bevorzugt wird jedoch, daß die gesamte Außenseite (Vorderseite) des vorderen Verbindungsstegs 10 und die gesamte Außenseite (Rückseite) des hinteren Verbindungsstegs 11 als Beschriftungsfeld herangezogen wird.

Die der Spritzenvorrichtung 1 zugeordnete Betätigungseinrichtung 20 ist durch eine gesonderte, einteilige Kolbenstangeneinheit 21 mit zwei Kolbenstangen 22, 23 gebildet, die an ihrem hinteren, in Fig.1 oberen, Ende einstückig durch einen gemeinsamen Griffteil 24 verbunden sind, an dessen Unterseite ein ebenfalls die Kolbenstangen 22, 23 miteinander verbindender, einstückig mitgeformter Versteifungssteg 25 anschließt. Der Griffteil 24 ist mit einer Griffmulde 26 für das Anlegen des Daumens beim Betätigen der Spritzenvorrichtung 1 versehen. Die Kolbenstangen 22, 23 sind beispielsweise, wie insbesondere aus Fig.4 ersichtlich ist, im Querschnitt kreiszylindrisch, wobei sie voll oder aber hohl sein können.

Die vorderen Enden der Kolbenstangen 22, 23 der Betätigungseinrichtung 20 werden bei Inbetriebnahme der Spritzenvorrichtung 1 an den Kolbenstopfen 28 bzw. 29 angesetzt, die bereits in den Spritzenkörpern 2, 3 nach deren Befüllen zwecks Verschließen eingesetzt wurden, wie nachstehend noch näher anhand der Fig.9 bis 11 erläutert werden wird.

Die Kolbenstangeneinheit 21 bildet nach der Spritzenvorrichtung 1 einen weiteren Teil der Spritzengarnitur, und zwar ohne die Kolbenstopfen 28, 29, die in der befüllten Spritzenvorrichtung 1 vorliegen, und sie wird vorzugsweise ebenfalls als Spritzgußteil, etwa aus ABS, hergestellt.

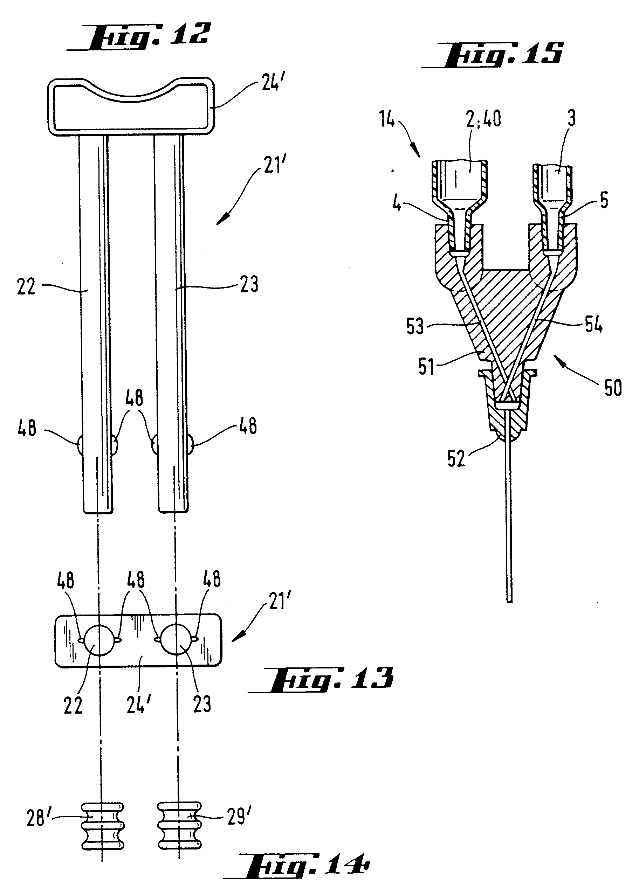

Die Kolbenstopfen 28, 29 können z.B. für eine Schnapp- oder Rastverbindung mit den vorderen Enden der Kolbenstangen 22, 23 ausgebildet sein, wie dies nachstehend noch näher anhand der Fig.7 erläutert werden soll, oder aber bevorzugt einfach aus vollem Material bestehen, an dem die Kolbenstangen 22, 23 beim Gebrauch zur Anlage kommen, wie nachstehend anhand der Fig.12 bis 14 erläutert werden wird.

Die Ausführungsform gemäß Fig.5 bis 8 entspricht größtenteils jener gemäß Fig.1 bis 4, so daß sich die nachfolgende Beschreibung dieser zweiten Ausführungsform vor allem auf die Unterschiede zwischen den beiden Ausführungsformen beschränken soll. Im übrigen sind in den Fig.5 bis 8 für die einzelnen Elemente dieselben Bezugszeichen wie für die entsprechenden Elemente der Ausführungsform gemäß Fig.1 bis 4 verwendet worden.

Bei der Ausführungsform gemäß Fig.5 bis 8 sind in der Kolbenstangeneinheit 21 Kolbenstangen 22', 23' mit einem sternförmigen Querschnitt vorgesehen, so daß beim Spritzgießen aufgrund der überall vergleichbaren Materialstärken ein einheitliches, gleichmäßiges Abkühlen und Erstarren des Materials sichergestellt ist. Im übrigen entspricht die Kolbenstangeneinheit 21 gemäß Fig.5 bis 8 jener gemäß Fig.1 und 4, wobei insbesondere wieder ein mit den Kolbenstangen 22', 23' einstückiger Griffteil 24 sowie ein Versteifungssteg 25 vorhanden sind. Hinsichtlich der weiteren Beschreibung der Kolbenstangeneinheit 21 kann daher auf die vorstehenden Erläuterungen verwiesen werden.

Aus Fig.7 ist sodann ersichtlich, daß die Kolbenstopfen, z.B. 28, eine hinterschnittene Bohrung 30 aufweisen können, die beim Zusammenstecken mit den Kolbenstangen, z.B. 22', mit einem an deren vorderem Ende vorgesehenen Widerhaken-artig verbreiterten Rastkopf 31 zusammenwirkt, so daß beim Aufdrücken der Kolbenstangen 22, 23 bzw. 22', 23' auf die Kolbenstopfen 28 bzw. 29 der Rastkopf 31 in die Bohrung 30 einschnappt.

Bei der Spritzeneinheit 14 gemäß Fig.5 und 6 ist sodann, anders als bei jener gemäß Fig.1 bis 4, ein einzelner, mittiger Verbindungssteg 32 vorgesehen, der allgemein entsprechend einer die Längsachsen der Spritzenkörper 2, 3 enthaltenden, in Fig.6 durch die strichpunktierte Linie 33 veranschaulichte Mittenebene verläuft. Zusätzlich oder anstattdessen können aber auch tangential an die Spritzenkörper 2, 3 angeschlossene Verbindungsstege 110, 111 - ähnlich den Verbindungsstegen 10, 11 in der Ausführungsform gemäß Fig.1 bis 4 - vorgesehen sein, wie in Fig.6 mit gestrichelten Linien veranschaulicht ist.

Auch bei der Spritzeneinheit 14 gemäß Fig.5 endet der mittige Verbindungssteg 32 wieder in Abstand vom hinteren Ende der Spritzen-einheit 14, wobei dieser Abstand vorzugsweise zumindest 5 mm, z.B. ungefähr 5 mm bis 15 mm, insbesondere 10 mm beträgt; dadurch ist wiederum eine Aufnahme-Ausnehmung 16 für die Einführung eines Schutzteils zwischen den Spritzenkörpern 2, 3 beim Befüllen der letzteren vorgesehen. Weiters ist auch zwischen den Konussen 4, 5 eine Ausnehmung 17 vorhanden.

Der Verbindungssteg 32 zwischen den Spritzenkörpern 2, 3 kann auch in verschiedene Beschriftungsfelder unterteilt sein, wie dies in Fig.5 mit gestrichelten Linien 35, 36 angedeutet ist, so daß beispielsweise ein oberes Beschriftungsfeld 37 und ein unteres Beschriftungsfeld 38, mit einem dazwischen vorgesehenen freien Feld 39, vorgesehen werden.

Sofern etwa aufgrund bestimmter, besonders erwünschter Lösungskonzentrationen der zu applizierenden Reaktionsflüssigkeiten ein von 1:1 abweichendes Mischungsverhältnis gewünscht wird, kann dies bei der vorliegenden Applikationsvorrichtung 1, insbesondere in der flachen, plattenförmigen Ausführung gemäß Fig.1 bis 4, wo eine konstante Querabmessung über die gesamte Vorrichtungsbreite erwünscht wäre, mit Vorteil dadurch erzielt werden, daß einer der Spritzenkörper, z.B. 2, mit einer von der Kreisform abweichenden Querschnittsform vorgesehen wird, etwa mit einer ovalen oder elliptischen Querschnittsform, wie dies in Fig.3 mit strichpunktierter Linie bei 40 angedeutet ist. Selbstverständlich könnte anstatt der dort angedeuteten größeren Breite auch eine vergleichsweise kleinere Spritzenkörper-Breite vorgesehen werden, wobei dann die Hauptachse der Ellipse des Querschnitts des Spritzenkörpers, z.B. 3, in Dickenrichtung der Spritzeneinheit 14 verläuft (nicht gezeigt).

In Fig.9, 9A und 9B ist schematisch das maschinelle Befüllen von Spritzeneinheiten 14 in einer Befüllungsanlage unter sterilen Bedingungen und unter Verhinderung einer gegenseitigen Kontamination der Spritzenkörper 2, 3 veranschaulicht. Im einzelnen sind in Fig.9 in einer schematischen Draufsicht in zwei Bereichen jeweils vier Spritzeneinheiten 14 gezeigt, von denen zuerst (s. Fig.9, linker Bereich, bzw. Fig. 9A) die einen Spritzenkörper 2 mit einer ersten Komponente gefüllt werden, wobei die anderen Spritzenkörper 3 mit einem U-förmig abgewinkelten Schutzteil 41, insbesondere einem U-förmigen Schutzblech, abgedeckt werden, der bzw. das in die entsprechenden Aufnahme-Ausnehmungen 16 eingeführt ist. Danach werden, vgl. in Fig.9 den rechten Bereich bzw. Fig.9B, die Spritzeneinheiten 14 mit der zweiten Komponente befüllt, wobei nun die einen Spritzenkörper 2, die bereits zuvor gefüllt wurden, mit einem winkelförmigen Schutzteil 42 abgedeckt werden, wogegen die zweite Komponente in die anderen, nunmehr nicht mehr abgedeckten Spritzenkörper 3 gefüllt wird. Zum Befüllen der Spritzenkörper 2, 3 können in Fig.9A und 9B nur ganz schematisch veranschaulichte Düsen 43 bzw. 44 vorgesehen werden.

Um das Befüllen sowie in der Folge die Aufbewahrung der befüllten Spritzeneinheit 1 zu ermöglichen, sind die Spritzenkonusse 4, 5 mit einer Verschlußkappe 45 verschlossen, vgl. Fig.9A, 9B und 10, wo diese Verschlußkappe 45 im Schnitt gezeigt ist.

In Fig.9 ist die Transportrichtung der Spritzeneinheiten 14 mit einem Pfeil 46 angedeutet. Dabei ist darauf hinzuweisen, daß selbstverständlich das Befüllen einerseits der Spritzenkörper 2 einer ersten Gruppe von Spritzeneinheiten 14, unter Abdeckung der Spritzenkörper 3 mit dem Schutzteil 41, und andererseits der Spritzenkörper 3 einer zweiten Gruppe von Spritzeneinheiten 14, unter Abdeckung der Spritzenkörper 2 mit dem Schutzteil 42, gleichzeitig erfolgen kann; die Befüllungsanlage kann dabei mit einer Transporteinrichtung, etwa einem intermittierend angetriebenen Kettenförderer oder dergl., wie an sich herkömmlich, ausgestattet sein, und diese Transporteinrichtung, die in Fig.9 nicht näher veranschaulicht ist, kann dabei taktweise, etwa um eine Strecke entsprechend jeweils vier Spritzeneinheiten 14, weiterbewegt werden, um so das aufeinanderfolgende Befüllen mit den beiden Komponenten unter Verhinderung einer gegenseitigen Kontamination zu bewerkstelligen. Die Schutzteile 41, 42 können an sich stationär an der Befüllungsanlage angebracht werden, sie können jedoch auch jeweils unmittelbar vor dem Befüllungsvorgang vertikal heranbewegt oder herangeschwenkt werden.

In Fig.10 ist sodann das sterile Verschließen der Spritzeneinheit 14, die zuvor wie beschrieben befüllt wurde, durch automatisches Einsetzen der Kolbenstopfen 28, 29 schematisch veranschaulicht. Die Kolbenstopfen 28, 29 werden dabei von oben mit irgendwelchen an sich herkömmlichen, maschinell bewegten Stempeln in die Spritzenkörper 2, 3 eingeführt.

Die so befüllte und verschlossene Spritzeneinheit 14 kann dann ohne Unterbrechung der das Befüllen und Verschließen beinhaltenden Linie steril verpackt und abtransportiert werden.

In Fig.11 ist eine Draufsicht auf die befüllte und gemäß Fig.10 verschlossene Spritzeneinheit 14 gezeigt. Dabei ist auch ersichtlich, daß bei dieser Spritzeneinheit 14, die im wesentlichen der Ausführungsform gemäß Fig.1 bis 4 entspricht, zwischen den Verbindungsstegen 10, 11 eine mit diesen einstückig geformte Querstrebe 47 vorgesehen sein kann, die zur zusätzlichen Versteifung und Festigkeitserhöhung der Spritzeneinheit 14 dient.

Aus Fig. 12 und 13 ist eine weitere, derzeit besonders bevorzugte Ausführungsform der Kolbenstangeneinheit 21' ersichtlich, wobei diese Kolbenstangeneinheit 21' mit den in Fig.14 gezeigten, aus vollem Material bestehenden Kolbenstopfen 28', 29' zusammenwirken soll.

Im einzelnen weist die Kolbenstangeneinheit 21' ähnlich wie jene gemäß Fig.1 und 3 zwei beispielsweise vollzylindrische, einige mm dicke Kolbenstangen 22, 23 auf, die an ihrem oberen, äußeren Ende wiederum einstückig über einen gemeinsamen Griffteil 24' miteinander verbunden sind.

Ein Unterschied bei der Kolbenstangeneinheit 21' gemäß Fig.12 und 13 im Vergleich etwa zu jener gemäß Fig.7 und 8 ist darin gelegen, daß die vorderen Stirnseiten der Kolbenstangen 22, 23 einfach plan abschließen und keine Verbindungsmittel für eine Rastverbindung oder dergl. mit den Kolbenstopfen 28', 29' gemäß Fig.14 aufweisen. Anstattdessen ist bei der Ausführungsform gemäß Fig.12 bis 14 vorgesehen, daß bei Ingebrauchnahme der befüllten und mit den Kolbenstopfen 28', 29' gemäß Fig.14 verschlossenen Spritzenvorrichtung 1, wenn die Kolbenstangeneinheit 21' gemäß Fig.12 und 13 am hinteren Ende der Spritzeneinheit 14 eingesetzt wird, die Kolbenstangen 22, 23 einfach an der äußeren Rückseite der Kolbenstopfen 28', 29' zur Anlage kommen. Auf diese Weise wird erreicht, daß die Kolbenstopfen 28', 29' nur in Vorwärtsrichtung, zum Herausdrücken der Komponenten des biologischen Mehrkomponentenmaterials aus der Spritzeneinheit 14, also in Richtung zu den Spritzenkonussen 4, 5 (siehe Fig.1) hin, bewegt werden können, nicht jedoch zurück bewegt werden können (beim Zurückziehen der Kolbenstangeneinheit 21' bewegen sich die Kolbenstangen 22, 23 von den Kolbenstopfen 28', 29' weg). So kann auf einfachste Weise verhindert werden, daß beim Gebrauch der Spritzenvorrichtung 1 unabsichtlich die Kolbenstopfen 28', 29' zurückgezogen werden, wobei bereits applizierter Gewebeklebstoff in die Vorrichtung eingesaugt würde, was ein Verstopfen der Vorrichtung zur Folge haben könnte.

An sich könnte dieser Effekt bei der Ausbildung gemäß Fig.7 und 8 auch dadurch erreicht werden, daß die dort gezeigte Rast- oder Schnappverbindung 30, 31 derart leicht ist, daß die Kolbenstopfen 28, 29 einen festeren Preßsitz in den Spritzenkörpern 2, 3 aufweisen als die Rastköpfe 31 in den Bohrungen 30; dadurch würden sich bei einem Zurückziehen der Kolbenstangeneinheit 21 die Rastköpfe 31 von den Kolbenstopfen 28, 29 lösen, wodurch ebenfalls ein Zurückziehen der Kolbenstopfen 28, 29 vermieden und die Bewegung der Kolbenstopfen 28, 29 auf bloß eine Vorwärtsbewegung beschränkt werden könnte.

Die Ausbildung gemäß Fig. 12 bis 14 wird ferner deshalb gegenüber jener gemäß Fig.7 und 8 bevorzugt, da beim Einsetzen der Kolbenstangeneinheit 21' keine Kraft auf die Kolbenstopfen 28', 29' ausgeübt werden muß, die zu einer unerwünschten Bewegung derselben in Richtung zu den Spritzenkonussen 4, 5 hin - die zu diesem Zeitpunkt noch mit Hilfe der Verschlußkappe 45 verschlossen sind - bzw. zu einer Druckerhöhung im Inneren der Spritzenkörper 2, 3 führen könnte. Die Folge hievon wäre, daß bei Abnehmen der Verschlußkappe 45 Komponentenmaterial bei den Spritzenkonussen ausgepreßt werden würde, was selbstverständlich unerwünscht wäre und nur durch ein vorheriges Zurückziehen der Kolbenstopfen vermieden werden könnte.

Andererseits wäre bei der vorliegenden Betätigungseinrichtung 20 ersichtlich eine nicht mit einer Druckerhöhung verbundene Schraubverbindung der Kolbenstangen mit den - bereits in die Spritzenkörper eingesetzten - Kolbenstopfen wegen der einstückigen Ausbildung der Kolbenstangeneinheit nicht möglich.

Um im Fall der Ausbildung der Kolbenstangeneinheit 21' gemäß Fig.12 und 13 ein unerwünschtes Herausfallen der Kolbenstangeneinheit 21' aus der jeweiligen Spritzeneinheit 14 zu verhindern (die Kolbenstangen 22, 23 können beispielsweise mit einem Spiel von ungefähr 0,5 mm im Inneren der Spritzenkörper 2, 3 beweglich sein), sind im Bereich der vorderen Enden der Kolbenstangen 22, 23 Flügel oder dergl. Vorsprünge angeformt, wie in Fig.12 und 13 bei 48 angedeutet ist. Diese Flügel 48 bewirken einen leichten Preß- oder Reibungssitz der Kolbenstangeneinheit 21' in den Spritzenkörpern 2, 3 bei ihrem Einsetzen in die jeweilige Spritzeneinheit 14, so daß die Kolbenstangeneinheit 21' ausreichend fest in der Spritzeneinheit 14 gehalten ist.

Die Kolbenstopfen 28', 29' bestehen vorzugsweise aus Silikonkautschukmaterial, um so adäquate Gleiteigenschaften ohne gesondertes Gleitmittel und auch nach langen Lagerzeiten (z.B. 1 bis 2 Jahre) sicherzustellen. Weiters ist dieses Silikonmaterial dann günstig, wenn die Spritzeneinheit mit einem Gewebeklebstoff befüllt wird, bei dem üblicherweise ein Gefriertrocknen vorgenommen wird, da hierdurch das Silikonmaterial hinsichtlich seiner Gleiteigenschaften nicht beeinträchtigt wird.

In Fig.15 ist der Vollständigkeit halber als Beispiel für den dritten Garniturteil der gesamten Spritzengarnitur, neben der Spritzeneinheit 14 und der Kolbenstangeneinheit 21 bzw. 21', ein Abgabeteil 50 in Form eines auf die Spritzenkonusse 4, 5 der Spritzenkörper 2, 3 einer Spritzeneinheit 14 (die nur teilweise in Fig. 15 dargestellt ist) aufsteckbaren Anschlußstückes 51 dargestellt, das in an sich herkömmlicher Weise eine Mischkanüle 52 trägt und mit gesonderten Förderkanälen oder -bohrungen 53, 54 für die verschiedenen Komponenten des in den Spritzenkörpern 2, 3 enthaltenen biologischen Mehrkomponentenmaterials vergehen ist. Ein solcher Mischkanülen-Abgabeteil ist z.B. aus der EP-A-37 393, EP-A-156 098 oder EP-A-210 160 bekannt; ein alternativer Abgabeteil wäre beispielsweise auch der aus der EP-A-37 393 bekannte Sprühkopf oder der aus der EP-A-156 098 bekannte Aufsteckteil mit abgeschlossenem Katheter.

In der Praxis kann ein derartiger Abgabeteil 50 zusammen mit der Kolbenstangeneinheit 21 bzw. 21' in einer gemeinsamen Verpackung verpackt sein, und gesondert hiervon wird die gefüllte und mit den Kolbenstopfen 28, 29 bzw. 28', 29' verschlossene Spritzeneinheit 14 (siehe Fig.10 und 11) verpackt und angeliefert. Selbstverständlich wäre es jedoch auch möglich, alle drei Garniturteile der beschriebenen Spritzengarnitur, d.h. die befüllte und mit dem Kolbenstopfen verschlossene Spritzeneinheit ebenso wie die Kolbenstangeneinheit und den Abgabeteil, in gesonderten Verpackungen steril verpackt vorzusehen.

Wenn die Erfindung vorstehend anhand von besonders bevorzugten Ausführungsbeispielen näher erläutert wurde, so sind doch selbstverständlich weitere Abwandlungen und Modifikationen im Rahmen der Erfindung möglich. Beispielsweise sind auch Spritzeneinheiten mit z.B. drei nebeneinander gemäß einer Ebene angeordneten, zueinander parallelen Spritzenkörpern denkbar, die untereinander durch Verbindungsstege wie beschrieben zu einer festen Spritzeneinheit verbunden sind. Weiters können die beschrieben Verbindungsstege, z.B. 10, 11, mit Verstärkungsrippen geformt sein, ebenso wie der Versteifungssteg 25 der Kolbenstangeneinheit 21.

Beim Befüllen der Spritzenkörper 2, 3 kann selbstverständlich auch anstatt des umgekehrt U-förmigen Schutzteils 41 bzw. 42 ein einfaches, ebenes, vertikales Schutzblech vorgesehen werden, das sich bis in die Höhe der jeweiligen Düse 43 bzw. 44 erstreckt und mit dem unteren Ende in die jeweilige Aufnahme-Ausnehmung (16 in Fig.1 und 5) der Spritzeneinheit 14 hineinragt, um so eine gegenseitige Kontamination mit der jeweils anderen Komponente zu verhindern.

## Patentansprüche

1. Spritzenvorrichtung zur Aufbewahrung und Applikation eines biologischen Mehrkomponentenmaterials, insbesondere Gewebeklebstoffes, mit mehreren durch einen Verbindungsteil (10, 11; 32; 110, 111) miteinander verbundenen parallelen, am vorderen Ende Konusse (4, 5) aufweisenden Spritzenkörpern (2, 3), vorzugsweise aus Kunststoffmaterial, für die verschiedenen Komponenten des Mehrkomponentenmaterials, wobei die Spritzenkörper (2, 3) mit dem Verbindungsteil (10, 11; 32; 110, 111) eine einstückige Spritzeneinheit (14) bilden, und wobei der Verbindungsteil in einem Abstand vom vorderen Ende endet, wodurch eine Ausnehmung (17) zwischen den Konussen (4, 5) vorhanden ist, dadurch gekennzeichnet, daß der Verbindungsteil (10, 11; 32; 110, 111) in einem Abstand von zumindest 5 mm von dem den Konussen (4, 5) entgegengesetzten Ende der Spritzeneinheit (14) endet, wodurch eine zur Aufnahme eines beim Befüllen abschirmenden Schutzteiles (41, 42) ausgebildete Ausnehmung (16) zwischen den Spritzenkörpern (2, 3) vorliegt.

2. Spritzenvorrichtung nach Anspruch 1, dadurch gekennzeichnet, daß der Verbindungsteil durch zumindest zwei zueinander parallele, plattenförmige Verbindungsstege (10, 11; 110, 111) gebildet ist.

3. Spritzenvorrichtung nach Anspruch 2, dadurch gekennzeichnet, daß jeder Verbindungssteg (10, 11) tangential an den jeweiligen Spritzenkörper (2, 3) anschließt.

4. Spritzenvorrichtung nach einem der Ansprüche 1 bis 3, dadurch gekennzeichnet, daß die Spritzeneinheit (14) flach und plattenförmig, mit einer Dicke entsprechend der Dicke bzw. dem Außendurchmesser der Spritzenkörper (2, 3), ausgebildet ist.

5. Spritzenvorrichtung nach Anspruch 3 oder 4, dadurch gekennzeichnet, daß die Spritzenkörper (2, 3) unterschiedliche Querschnittsflächen aufweisen und die Querschnittsfläche (40) zumindest eines Spritzenkörpers (2) oval ist, wobei ihre Abmessung quer zur Dickenrichtung der plattenförmigen Spritzeneinheit (14) von jener der Querschnittsfläche des oder der anderen Spritzenkörper (3) verschieden ist.

6. Spritzenvorrichtung nach einem der Ansprüche 1, 4 oder 5, dadurch gekennzeichnet, daß der Verbindungsteil durch zumindest einen plattenförmigen Verbindungssteg (32; 110, 111) gebildet ist, der in der durch die Längsachsen der Spritzenkörper (2, 3) definierten Mittenebene (33) angeordnet und mittig an den jeweiligen Spritzenkörper (2, 3) angeschlossen ist.

7. Spritzenvorrichtung nach wenigstens einem der Ansprüche 1 bis 6, dadurch gekennzeichnet, daß der Verbindungsteil (10, 11; 32; 110, 111) in einem Abstand von höchstens 15 mm vom den Konussen entgegengesetzten Ende der Spritzeneinheit (14) endet.

8. Spritzenvorrichtung nach Anspruch 7, dadurch gekennzeichnet, daß der Verbindungsteil (10, 11; 32; 110, 111) in einem Abstand von 10 mm vom den Konussen entgegengesetzten Ende der Spritzeneinheit (14) endet.

9. Spritzenvorrichtung nach einem der Ansprüche 1 bis 7, dadurch gekennzeichnet, daß an den den Konussen entgegengesetzten Enden der Spritzenkörper (2, 3) Fingergriffe (8, 9) angeformt sind.

10. Spritzenvorrichtung nach einem der Ansprüche 1 bis 9, dadurch gekennzeichnet, daß zumindest ein Verbindungsteil (10, 11) wenigstens ein Beschriftungsfeld (18, 19; 37, 38) aufweist.

11. Spritzenvorrichtung nach einem der Ansprüche 1 bis 10, dadurch gekennzeichnet, daß die Spritzeneinheit (14) zumindest bereichsweise, z.B. in Form eines Streifens, röntgendichtes Material aufweist.

12. Spritzenvorrichtung nach einem der Ansprüche 1 bis 11, dadurch gekennzeichnet, daß die Spritzenkörper (2, 3) befüllt und durch gesonderte, in sie eingesetzte Kolbenstopfen (28, 29; 28', 29') verschlossen sind.

13. Spritzenvorrichtung nach Anspruch 12, dadurch gekennzeichnet, daß die Kolbenstopfen (28, 29; 28', 29') mittels Kolbenstangen (22, 23; 22', 23') nur in Richtung der Konusse verschiebbar gelagert sind.

14. Spritzenvorrichtung nach Anspruch 12 oder 13, dadurch gekennzeichnet, daß die Kolbenstopfen (28', 29') aus Silikonmaterial hergestellt sind.

15. Spritzenvorrichtung nach einem der Ansprüche 12 bis 14, dadurch gekennzeichnet, daß die Kolbenstopfen (28, 29; 28', 29') für einen Eingriff mit Kolbenstangen (22, 23; 22', 23') einer einstückigen Kolbenstangeneinheit (21) angeordnet sind, die einen gemeinsamen, mit den Kolbenstangen einstückigen Griffteil (24) aufweist, und daß ein gesonderter Abgabeteil (50) zum Aufstecken auf die Konusse (4, 5) der Spritzenkörper (2, 3) vorgesehen ist.

16. Spritzenvorrichtung nach Anspruch 15, dadurch gekennzeichnet, daß die Kolbenstangeneinheit (21) zumindest bereichsweise, z.B. in Form eines Streifens, röntgendichtes Material aufweist.

17. Spritzenvorrichtung nach Anspruch 15 oder 16, dadurch gekennzeichnet, daß der Abgabeteil (50) zumindest bereichsweise, z.B. in Form eines Streifens, röntgendichtes Material aufweist.

18. Spritzenvorrichtung nach einem der Ansprüche 15 bis 17, dadurch gekennzeichnet, daß der Griffteil (24) mit einem sich zwischen den Kolbenstangen (22, 23; 22', 23') erstreckenden, mit ihnen fest verbundenen Versteifungssteg (25) versehen ist.

19. Spritzenvorrichtung nach einem der Ansprüche 15 bis 18, dadurch gekennzeichnet, daß die Kolbenstangeneinheit (21) ein einstückiger Spritzgußteil ist.

20. Spritzenvorrichtung nach einem der Ansprüche 15 bis 19, dadurch gekennzeichnet, daß die Kolbenstangeneinheit (21) aus Acrylnitril-Butadien-Styrol-Copolymer (ABS) besteht.

21. Verfahren zum Herstellen einer befüllten sterilen Spritzenvorrichtung nach einem der Ansprüche 1 bis 20, wobei die Spritzenkörper einer sterilen Spritzeneinheit in einem Stück steril hergestellt und gehalten oder nach ihrer Herstellung sterilisiert werden, dadurch gekennzeichnet, daß die die Konusse aufweisenden Spritzenkörper-Enden der sterilen Spritzeneinheit dicht verschlossen werden, sodann ein den oder die jeweils anderen Spritzenkörper abschirmender Schutzteil in die Ausnehmung zwischen den (jeweiligen) Spritzenkörpern an deren den Konussen entgegengesetzten Enden eingeführt wird und die Spritzenkörper in einer Befüllungsanlage maschinell und unter sterilen Bedingungen mit den Komponenten des biologischen Mehrkomponentenmaterials befüllt und anschließend mit sterilen Kolbenstopfen verschlossen werden.

22. Verfahren nach Anspruch 21, dadurch gekennzeichnet, daß die Spritzenvorrichtung unmittelbar nach dem Befüllen mit den Komponenten und Verschließen mit den Kolbenstopfen steril verpackt wird.

23. Verfahren nach Anspruch 21 oder 22, dadurch gekennzeichnet, daß jeweils die einander entsprechenden Spritzenkörper mehrerer in einer Reihe angeordneter Spritzeneinheiten gleichzeitig unter Einführung (jeweils) eines gemeinsamen Schutzteiles in die Ausnehmungen dieser Spritzeneinheiten steril befüllt werden.

## Claims

1. A syringe device for storing and applying a biological multi-component material, in particular a tissue adhesive, comprising several parallel syringe bodies (2, 3), preferably made of synthetic material, for the different components of the multi-component material, the syringe bodies (2, 3) being interconnected by a connection part (10, 11; 32; 110, 111) and including coni (4, 5) at their front ends, the syringe bodies (2, 3) forming a one-piece syringe unit (14) with the connection part (10, 11; 32; 110, 111), and the connection part terminating at a distance from the front end so as to leave a recess (17) between the coni (4, 5), characterised in that the connection part (10, 11; 32; 110, 111) terminates at a distance of at least 5 mm from that end of the syringe unit (14) which is located opposite the coni (4, 5) so as to leave a recess (16) between the syringe bodies (2, 3) formed to accommodate a protection member (41, 42) constituting a shield during filling.

2. A syringe device according to claim 1, characterised in that the connection part is comprised of at least two parallel plate-shaped connection webs (10, 11; 110, 111).

3. A syringe device according to claim 2, characterised in that each connection web (10, 11) tangentially joins the respective syringe body (2, 3).

4. A syringe device according to any one of claims 1 to 3, characterised in that the syringe unit (14) is flat and plate-shaped, having a thickness corresponding to the thickness or to the external diameter, respectively, of the syringe bodies (2, 3).

5. A syringe device according to claim 3 or 4, charcterised in that the syringe bodies (2, 3) have different cross sectional areas and that the cross sectional area (40) of at least one syringe body (2) is oval, its dimension transverse to the thickness direction of the plate-shaped syringe unit (14) being different from that of the cross sectional area of the other one(s) of the syringe bodies (3).

6. A syringe device according to any one of claims 1, 4 or 5, characterised in that the connection part is formed by at least one plate-shaped connection web (32; 110, 111) which is arranged in the median plane (33) defined by the longitudinal axes of the syringe bodies (2, 3) and adjoins centrally to the respective syringe body (2, 3).

7. A syringe device according to at least one of claims 1 to 6, characterised in that the connection part (10, 11; 32; 110, 111) terminates at a distance of 15 mm at the most from that end of the syringe unit (14) which is located opposite the coni.

8. A syringe device according to claim 7, characterised in that the connection part (10, 11; 32; 110, 111) terminates at a distance of 10 mm from that end of the syringe unit (14) which is located opposite the coni.

9. A syringe device according to any one of claims 1 to 7, characterised in that finger grips (8, 9) are molded to those ends of the syringe bodies (2, 3) which are located opposite the coni.

10. A syringe device according to any one of claims 1 to 9, characterised in that at least one connection part (10, 11) comprises at least one inscription area (18, 19; 37, 38).

11. A syringe device according to any one of calims 1 to 10, characterised in that the syringe unit (14) comprises X-ray-tight material at least in regions thereof, e.g. in the form of a strip.

12. A syringe device according to any one of claims 1 to 11, characterised in that the syringe bodies (2, 3) are filled and are closed by separate piston plugs (28, 29; 28', 29') inserted thereinto.

13. A syringe device according to claim 12, characterised in that the piston plugs (28, 29; 28', 29') are mounted to be displaceable only in the direction towards the coni by means of piston rods (22, 23; 22', 23').

14. A syringe device according to claim 12 or 13, characterised in that the piston plugs (28', 29') are made of silicone material.

15. A syringe device according to any one of claims 12 to 14, characterised in that the piston plugs (28, 29; 28', 29') are arranged for engagement with poston rods (22, 23; 22', 23') of a one-piece piston rod unit (21) which has a common grip element (24) integral with the piston rods, and that a separate dispensing element (50) is provided to be slipped onto the coni (4, 5) of the syringe bodies (2, 3).

16. A syringe device according to claim 15, characterised in that the piston rod unit (21) comprises X-ray-tight material at least in regions thereof, e.g. in the form of a strip.

17. A syringe device according to claim 15 or 16, characterised in that the dispensing element (50) comprises X-ray-tight material at least in regions thereof, e.g. in the form of a strip.

18. A syringe device according to any one of claims 15 to 17, characterised in that the grip element (24) is provided with a reinforcement web (25) extending between the piston rods (22, 23; 22', 23') and fixedly connected thereto.

19. A syringe device according to any one of claims 15 to 18, characterised in that the piston rod unit (21) is a one-piece injection-molded part.

20. A syringe device according to any one of claims 15 to 19, characterised in that the piston rod unit (21) is made of acrylonitrile-butadiene-styrene copolymer (ABS).

21. A method of producing a filled sterile syringe device according to any one of claims 1 to 20, wherein the syringe bodies of a sterile syringe unit are sterilely produced in one piece and kept sterile or are sterilized after their production, characterised in that the coni-equipped syringe body ends of the sterile syringe unit are sealingly closed, whereupon a protection member shielding off the respective other syringe body or several respective other syringe bodies is inserted into the recess between the (respective) syringe bodies at their ends located opposite the coni, and the syringe bodies are machine-filled in a filling station under sterile conditions with the components of the biological multi-component material and subsequently are closed with sterile piston plugs.

22. A method according to claim 21, characterised in that the syringe device is sterilely packed immediately after having been filled with the components and having been closed with the piston plugs.

23. A method according to claim 21 or 22, characterised in that the corresponding syringe bodies of several syringe units arranged in a row are sterilely filled simultaneosly while introducing a (respective) common protection member into the recesses of these syringe units.

## Revendications

1. Dispositif à seringues pour conserver et appliquer un matériau biologique à plusieurs composants, en particulier un histoadhésif avec plusieurs corps de seringues (2, 3) reliés entre eux par une partie de liaison (10, 11; 32; 110, 111), parallèles, comportant des cônes (4,5) à l'extrémité avant, de préférence en matériau à base de matière synthétique, pour les différents composants du matériau à plusieurs composants, où les corps de seringues (2, 3) forment avec la partie de liaison (10, 11; 32; 110, 111) une unité de seringues d'une pièce (14), et où la partie de liaison se termine à une distance de l'extrémité avant, de sorte qu'il existe un évidement (17) entre les cônes (4, 5), caractérisé en ce que la partie de liaison (10, 11; 32; 110, 111) se termine à une distance d'au moins 5 mm de l'extrémité de l'unité de seringues (14) opposée aux cônes (4, 5) de sorte qu'il existe entre les corps de seringues (2,3) un évidement (16) agencé pour recevoir une partie protectrice (41, 42) formant écran lors du remplissage.

2. Dispositif à seringues selon la revendication 1, caractérisé en ce que la partie de liaison est formée par au moins deux traverses de liaison en forme de plaques (10, 11; 110, 111) paralléles entre elles.

3. Dispositif à seringues selon la revendication 2, caractérisé en ce que chaque traverse de liaison (10, 11) se raccorde tangentiellement aux différents corps de seringues (2, 3).

4. Dispositif à seringues selon l'une des revendications 1 à 3, caractérisé en ce que l'unité de seringues (14) est plate et en forme de plaque, avec une épaisseur correspondant à l'épaisseur ou au diamètre externe des corps de seringues (2, 3).

5. Dispositif à seringues selon la revendication 3 ou 4, caractérisé en ce que les corps de seringues (2, 3) présentent des surfaces en section droite différentes et la surface en section droite (40) d'au moins un corps de seringue (2) est ovale, sa dimension transversalement à la direction d'épaisseur de l'unité de seringues en forme de plaque (14) étant différente de celle de la surface en section droite du ou des autres corps de seringues (3).

6. Dispositif à seringues selon l'une des revendications 1, 4 ou 5, caractérisé en ce que la partie de liaison est formée par au moins une traverse de liaison en forme de plaque (32 ; 110, 111) qui est agencée dans le plan médian (33) défini par les axes longitudinaux des corps de seringues (2, 3) et qui est raccordée de manière médiane aux différents corps de seringues (2,3).

7. Dispositif à seringues selon au moins l'une des revendications 1 à 6, caractérisé en ce que la partie de liaison (10, 11; 32; 110, 111) se termine à une distance d'au plus 15 mm de l'extrémité de l'unité de seringues (14) qui est opposée aux cônes.

8. Dispositif à seringues selon la revendication 7, caractérisé en ce que la partie de liaison (10, 11 ; 32 ; 110, 111) se termine à une distance de 10mm de l'extrémité de l'unité de seringues (14) qui est opposée aux cônes.

9. Dispositif à seringues selon l'une des revendications 1 à 7, caractérisé en ce que des poignées (8, 9) sont formées au niveau des extrémités des corps de seringues (2, 3) opposées aux cônes.

10. Dispositif à seringues selon l'une des revendications 1 à 9, caractérisé en ce qu'au moins une partie de liaison (10, 11) comporte au moins un domaine d'inscription (18, 19 ; 37, 38).

11. Dispositif à seringues selon l'une des revendications 1 à 10, caractérisé en ce que l'unité de seringues (14) comporte un matériau opaque aux rayons X, selon au moins un ou des domaines, par exemple sous forme d'une bande.

12. Dispositif à seringues selon l'une des revendications 1 à 11, caractérisé en ce que les corps de seringues (2, 3) sont remplis et sont fermés par des bouchons-pistons séparés (28, 29 ; 28', 29') insérés dans ceux-ci.

13. Dispositif à seringues selon la revendication 12, caractérisé en ce que les bouchons-pistons (28, 29 ; 28', 29') sont positionnés à l'aide de tiges de pistons (22, 23 ; 22', 23') en n'étant déplaçables qu'en direction des cônes.

14. Dispositif à seringues selon la revendication 12 ou 13, caractérisé en ce que les bouchons-pistons (28', 29') sont fabriqués en matériau à base de silicone.

15. Dispositif à seringues selon l'une des revendications 12 à 14, caractérisé en ce que les bouchons-pistons (28, 29; 28', 29') sont agencés en vue d'être actionnés avec des tiges de pistons (22, 23; 22', 23') d'une unité de tiges de pistons d'une pièce (21) qui comporte une poignée commune (24) d'une pièce avec les tiges de pistons et en ce qu'une partie d'émission séparée (50) est prévue pour être emboîtée sur les cônes (4,5) des corps de seringues (2, 3).

16. Dispositif à seringues selon la revendication 15, caractérisé en ce que l'unité de tiges de pistons (21) comporte un matériau opaque aux rayons X, au moins suivant un ou des domaines, par exemple sous forme d'une bande.

17. Dispositif à seringues selon la revendication 15 ou 16, caractérisé en ce que la partie d'émission (50) comporte un matériau opaque aux rayons X, au moins selon un ou des domaines, par exemple sous forme d'une bande.

18. Dispositif à seringues selon l'une des revendications 15 à 17, caractérisé en ce que la poignée (24) est munie d'une traverse de renforcement (25) qui s'étend entre les tiges de pistons (22, 23 ; 22', 23') et qui est reliée à celles-ci de manière fixe.

19. Dispositif à seringues selon l'une des revendications 15 à 18, caractérisé en ce que l'unité de tiges de pistons (21) est une partie moulée par injection d'une pièce.

20. Dispositif à seringues selon l'une des revendications 15 à 19, caractérisé en ce que l'unité de tiges de pistons (21) consiste en copolymère acrylonitrile-butadiène-styrène (ABS).

21. Procédé de fabrication d'un dispositif à seringues stérile rempli selon l'une des revendications 1 à 20, où les corps de seringues d'une unité de seringues stérile sont fabriqués et maintenus d'une pièce dans les conditions stériles ou sont stérilisés après leur fabrication, caractérisé en ce que les extrémités des corps de seringues de l'unité de seringues stérile qui comportent les cônes sont fermées de manière étanche, après quoi une partie protectrice protégeant le ou les autres corps de seringues est insérée dans l'évidement entre les (différents) corps de seringues au niveau de leurs extrémités opposées aux cônes et les corps de seringues sont remplis à la machine dans une installation de remplissage et dans des conditions stériles avec les composants du matériau biologique à plusieurs composants puis sont fermés avec des bouchons-pistons stériles.

22. Procédé selon la revendication 21, caractérisé en ce que le dispositif à seringues est emballé dans des conditions stériles immédiatement après le remplissage avec les composants et la fermeture avec les bouchonspistons.

23. Procédé selon la revendication 21 ou 22, caractérisé en ce que, dans chaque cas, les corps de seringues mutuellement correspondants de plusieurs unités de seringues disposées en une rangée sont remplis simultanément dans des conditions stériles avec insertion (dans chaque cas) d'une partie protectrice commune dans les évidements de ces unités de seringues.
